# EUROPEAN PATENT APPLICATION

(11) **EP 2 592 419 A1**
(43) Date of publication of application: **15.05.2013**
(21) Application number: 11803572.4
(22) Date of filing: 05.07.2011
(51) Int. Cl.: G01N 33/569, C07K 14/045, G01N 33/53

(54) **METHOD FOR DETECTION OF INFECTION WITH HUMAN CYTOMEGALOVIRUS**

(30) Priority: 07.07.2010 JP 2010154937
(71) Applicant: FUJIREBIO INC., Shinjuku-ku, Tokyo 163-0410 (JP)
(72) Inventor: FUJII, Nobuyuki, Tokyo 103-0007 (JP); HONDA, Hideo, Tokyo 103-0007 (JP); UCHIDA, Yoshiaki, Tokyo 103-0007 (JP); OMI, Kazuya, Tokyo 103-0007 (JP)
(74) Representative: Keirstead, Tanis Evelyne
(86) International application number: PCT/JP2011/065343
(87) International publication number: WO 2012/005238

(57) **Abstract**

A novel means by which HCMV infection can be detected with a high sensitivity and whose practicality is high is disclosed. The present inventors synthesized as many as 15 kinds of HCMV proteins in their full length forms, and intensively studied their reactivities with sera from HCMV-infected patients to find that all the infected patients can be detected without fail when using the pp28 full length protein as an antigen. The pp28 full length protein can be synthesized and purified as a recombinant protein in a large scale by using *Escherichia coli,* and can be commercially used as an antigen for HCMV tests.

SEQUENCE LISTING

<110> FUJIREBIO Inc.

<120> Method for Detection of Infection with Human Cytomegalovirus

<130> TEK/FP6873236

<140> EP11803572.4
<141> 2011-07-05

<150> JP2010-154937
<151> 2010-07-07

<150> PCT/JP2011/065343
<151> 2011-07-05

<160> 4

<170> PatentIn version 3.1

<210> 1
<211> 573
<212> DNA
<213> Human cytomegalovirus

<220>
<221> CDS
<222> (1)..(573)
<223>

<400> 1
atg ggt gcc gaa ctc tgc aaa cga ata tgt tgt gag ttc ggt acc acg 48
Met Gly Ala Glu Leu Cys Lys Arg Ile Cys Cys Glu Phe Gly Thr Thr
1               5                   10                  15

ccc ggt gag ccc ctg aaa gat gct ctg ggt cgc cag gtg tct cta cgc 96
Pro Gly Glu Pro Leu Lys Asp Ala Leu Gly Arg Gln Val Ser Leu Arg
            20                  25                  30

tcc tac gac aac atc cct ccg act tcc tcc tcg gac gaa ggg gag gac 144
Ser Tyr Asp Asn Ile Pro Pro Thr Ser Ser Ser Asp Glu Gly Glu Asp
        35                  40                  45

gat gac gac ggg gag gat gac gat aac gag gag cgg caa cag aag ctg 192
Asp Asp Asp Gly Glu Asp Asp Asp Asn Glu Glu Arg Gln Gln Lys Leu
    50                  55                  60

cgg ctc tgc ggt agt ggc tgc ggg gga aac gac agt agt agc ggc agc 240
Arg Leu Cys Gly Ser Gly Cys Gly Gly Asn Asp Ser Ser Ser Gly Ser
65                  70                  75                  80

cac cgc gag gcc acc cac gac ggc tcc aag aaa aac gcg gtg cgc tcg 288
His Arg Glu Ala Thr His Asp Gly Ser Lys Lys Asn Ala Val Arg Ser
                85                  90                  95

acg ttt cgc gag gac aag gct ccg aaa ccg agc aag cag tca aaa aag 336
Thr Phe Arg Glu Asp Lys Ala Pro Lys Pro Ser Lys Gln Ser Lys Lys
            100                 105                 110

aaa aag aaa ccc tca aaa cat cac cac cat cag caa agc tcc att atg 384
Lys Lys Lys Pro Ser Lys His His His His Gln Gln Ser Ser Ile Met
        115                 120                 125

cag gag acg gac gac cta gac gaa gag gac acc tca att tac ctg tcc 432

Gln Glu Thr Asp Asp Leu Asp Glu Glu Asp Thr Ser Ile Tyr Leu Ser
    130                 135                 140

ccg ccc ccg gtc ccc ccc gtc cag gtg gtg gct aag cga ctg ccg cgg      480
Pro Pro Pro Val Pro Pro Val Gln Val Val Ala Lys Arg Leu Pro Arg
145                 150                 155                 160

ccc gac aca ccc agg act ccg cgc caa aag aag att tca caa cgt cca      528
Pro Asp Thr Pro Arg Thr Pro Arg Gln Lys Lys Ile Ser Gln Arg Pro
                165                 170                 175

ccc acc ccc ggg aca aaa aag ccc gcc gcc tcc ttg ccc ttt taa          573
Pro Thr Pro Gly Thr Lys Lys Pro Ala Ala Ser Leu Pro Phe
            180                 185                 190

<210>  2
<211>  190
<212>  PRT
<213>  Human cytomegalovirus

<400>  2

Met Gly Ala Glu Leu Cys Lys Arg Ile Cys Cys Glu Phe Gly Thr Thr
1               5                   10                  15

Pro Gly Glu Pro Leu Lys Asp Ala Leu Gly Arg Gln Val Ser Leu Arg
            20                  25                  30

Ser Tyr Asp Asn Ile Pro Pro Thr Ser Ser Ser Asp Glu Gly Glu Asp
        35                  40                  45

Asp Asp Asp Gly Glu Asp Asp Asp Asn Glu Glu Arg Gln Gln Lys Leu
    50                  55                  60

Arg Leu Cys Gly Ser Gly Cys Gly Gly Asn Asp Ser Ser Ser Gly Ser
65                  70                  75                  80

His Arg Glu Ala Thr His Asp Gly Ser Lys Lys Asn Ala Val Arg Ser
                85                  90                  95

Thr Phe Arg Glu Asp Lys Ala Pro Lys Pro Ser Lys Gln Ser Lys Lys
            100                 105                 110

Lys Lys Lys Pro Ser Lys His His His His Gln Gln Ser Ser Ile Met
        115                 120                 125

Gln Glu Thr Asp Asp Leu Asp Glu Glu Asp Thr Ser Ile Tyr Leu Ser
    130                 135                 140

Pro Pro Pro Val Pro Pro Val Gln Val Val Ala Lys Arg Leu Pro Arg
145                 150                 155                 160

Pro Asp Thr Pro Arg Thr Pro Arg Gln Lys Lys Ile Ser Gln Arg Pro
                165                 170                 175

Pro Thr Pro Gly Thr Lys Lys Pro Ala Ala Ser Leu Pro Phe
            180                 185                 190

<210>  3
<211>  31
<212>  DNA
<213>  Artificial

<220>
<223>  primer for amplification of pp28 gene

<400>  3
agtggatcca tgggtgccga actctgcaaa c                                    31

<210>  4
<211>  30
<212>  DNA
<213>  Artificial

<220>
<223>  primer for amplification of pp28 gene

<400>  4
agagcggccg cttaaaaggg caaggaggcg                                      30

## Description

### TECHNICAL FIELD

The present invention relates to a method for detecting human cytomegalovirus infection.

### BACKGROUND ART

In serodiagnosis of an infectious disease caused by human cytomegalovirus (HCMV), viral antigens (native antigens) have been generally used. However, in the case of native antigens, lot differences caused due to the preparation methods are not negligible. In addition, there are problems in safety and handiness, since a virus itself is to be handled.

In order to achieve standardization of the serodiagnosis of HCMV, it is desired to establish recombinant antigens. However, it is known that the sensitivity and the specificity of the test is insufficient when recombinant antigens are used alone. This is because it is difficult to select from a large number of viral antigens an appropriate antigen which should be produced as a recombinant. In addition thereto, the fact that partially expressed fragments of the individual antigen proteins are used is also considered to be one cause thereof.

For example, pp150, which is known as a major antigen, is a protein that has a molecular weight of about 150 kDa, and therefore cannot be synthesized in a large scale by using *Escherichia coli;* and it is difficult to utilize its full length protein for the tests. Hence, in commercially available test kits, a fragment of pp150 (typically, about 30 to 40 residues) is appropriately mixed with other HCMV protein fragments, and the mixture is used as an antigen. However, it has been reported that, when such partially expressed fragments are used alone, the sensitivity of the test is about 60%, which is insufficient.

However, in the field of serologic tests by immunoassay, it is also known that the detection sensitivity is not always increased even though a full length antigen is used. For example, in Non-patent Document 1, HCMV-positive patient sera were examined for their antibody reactivities with HCMV native antigens (which contain the viral proteins in full length form), and the antibody reactivities with the individual proteins were each evaluated. According to this document, it is only pp150 that could attain a detection sensitivity of approximately 100%, and some of the proteins showed a detection sensitivity of about 50% or less, which is very low.

### PRIOR ART DOCUMENTS

### PATENT DOCUMENTS

Patent Document 1: JP 2000-514300 A

### NON-PATENT DOCUMENTS

Non-patent Document 1: Journal of Clinical Microbiology: 37(1), pp. 179-188, 1999

### SUMMARY OF THE INVENTION

### PROBLEMS TO BE SOLVED BY THE INVENTION

An object of the present invention is to provide a novel means by which HCMV infection can be detected with a high sensitivity and whose practicality is high.

### MEANS FOR SOLVING THE PROBLEMS

The present inventors synthesized as many as 15 kinds of HCMV proteins in their full length forms, and intensively studied their reactivities with sera from HCMV-infected patients to find that all the infected patients can be detected without fail when using the pp28 full length protein as an antigen, and that the pp28 full length protein can be synthesized and purified as a recombinant protein in a large scale by using *Escherichia coli,* and can be commercially used as an antigen for HCMV tests, thereby completing the present invention.

That is, the present invention provides a method for detecting human cytomegalovirus infection, comprising measuring an antibody against pp28 which may be contained in a sample separated from a subject by immunoassay using as an antigen an artificial polypeptide composed of an amino acid sequence having an identity of not less than 90% to the amino acid sequence shown in SEQ ID NO:2, said artificial polypeptide having a reactivity to bind by antigen-antibody reaction to an antibody induced in a living body against pp28 produced by human cytomegalovirus. The present invention also provides a reagent for detecting human cytomegalovirus infection, comprising an artificial polypeptide composed of an amino acid sequence having an identity of not less than 90% to the amino acid sequence shown in SEQ ID NO:2, said artificial polypeptide having a reactivity to bind by antigen-antibody reaction to an antibody induced in a living body against pp28 produced by human cytomegalovirus. The present invention further provides a kit for detecting human cytomegalovirus infection, which comprises the above-described reagent of the present invention.

### EFFECTS OF THE INVENTION

By the present invention, a means for detecting HCMV infection which has an extremely excellent detection sensitivity was provided. According to the present invention, all HCMV-infected patients can be surely detected by a simple immunoassay without fail. As a known antibody testing method, a method in which a pp28 fragment is used as an antigen has been known. For example, also in Non-patent Document 1, a method in which pp28 is used in a form of a fragment having a size of about 30 residues to detect an anti-HCMV antibody in serum is described. However, according to Non-patent Document 1, false negatives occurred in not less than 10% of patients when the pp28 fragment is used. False negative is a serious problem in clinical tests since infected patients are to be missed if it occurs. By the method of the present invention, infected patients will be able to be surely detected without missing, thereby contributing greatly to clinical tests.

### BRIEF DESCRIPTION OF THE DRAWINGS

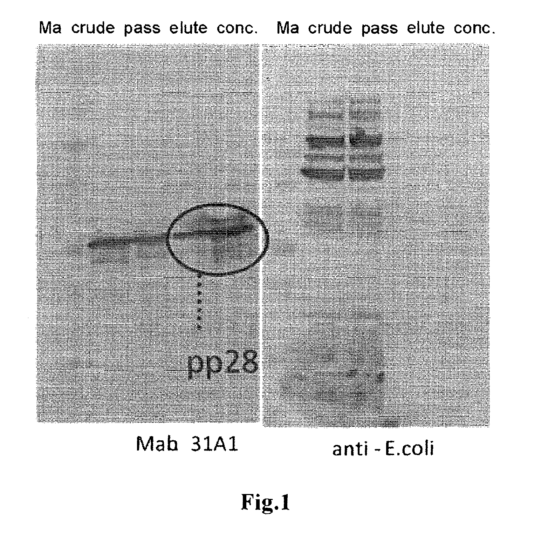

Fig. 1 shows the results of confirmation by Western blotting of the process of purification of a recombinant pp28 protein expressed in *Escherichia coli* with an antibody column. The left panel shows the results of detection by using the anti-pp28 monoclonal antibody prepared in the Examples, and the right panel shows the results of detection by using an antibody against an *Escherichia coli* antigen. Ma: marker, crude: before addition to the column, pass: passed-through fraction, elute: eluted fraction, and conc.: concentrated fraction.

### MODE FOR CARRYING OUT THE INVENTION

pp28 is a 28 kDa phosphoprotein which is one of the HCMV structural proteins, and may also be referred to as UL99 because it is encoded by *UL99* gene. The amino acid sequence shown in SEQ ID NO:2 is the amino acid sequence of pp28 which HCMV AD169 strain has, and is also registered in GenBank under the accession No. X17403 (complete genome) and so on. The base sequence of a viral DNA encoding the amino acid sequence shown in SEQ ID NO:2 is shown in SEQ ID NO:1.

In the present invention, an artificial pp28 composed of the amino acid sequence shown in SEQ ID NO:2 is used as an antigen. Alternatively, an artificial polypeptide which is composed of the same amino acid sequence as that shown in SEQ ID NO:2 except that a small number of the amino acid residues are substituted, deleted, inserted and/or added, and which has a reactivity to bind by antigen-antibody reaction to an antibody induced in a living body against pp28 produced by HCMV, is used as an antigen. The latter polypeptide has an identity of not less than 90%, preferably not less than 95%, more preferably not less than 98% to SEQ ID NO:2. As the latter polypeptide, one composed of the same amino acid sequence as that shown in SEQ ID NO:2 except that one or several amino acid residues are substituted, deleted, inserted and/or added is also preferred.

In the Examples, all of 100 specimens from HCMV-infected patients were able to be detected by using only a pp28 full length protein composed of the amino acid sequence shown in SEQ ID NO:2 as an antigen. Viruses undergo genomic mutations frequently, and various types of mutated sequences of the pp28 amino acid sequence have been discovered in clinical isolates. Accordingly, it is considered that there may be some differences in the HCMV pp28 amino acid sequence among the 100 infected patients from which these specimens were taken. Even in the case of antibodies which have been induced in the living bodies against the naturally-occurring pp28s composed of such various amino acid sequences, all of such antibodies react with the polypeptide composed of the amino acid sequence shown in SEQ ID NO:2 and are thus detected, and therefore it is considered that an antibody against pp28 (an anti-pp28 antibody) can also be detected similarly when using as an antigen a polypeptide whose amino acid sequence is partially different from SEQ ID NO:2. It is highly probable that the reactivity of a given polypeptide with an anti-pp28 antibody induced in an HCMV-infected person is at a similar level to that of pp28 composed of the original sequence shown in SEQ ID NO:2 as long as the polypeptide has a sufficiently-high identity to SEQ ID NO:2.

Whether a polypeptide having an arbitrary amino acid sequence has a reactivity to bind by antigen-antibody reaction to an antibody induced in a living body against pp28 produced by HCMV can be easily confirmed, for example, by reacting the polypeptide with a sample such as serum separated from a patient known to be infected with HCMV. If the binding between an antibody present in the serum and the polypeptide can be confirmed, the polypeptide can be judged to have the above-mentioned reactivity.

As used herein, the term "identity" of amino acid sequences means a value expressed in percentage which is calculated by aligning two amino acid sequences to be compared such that as many amino acid residues as possible are matched and then dividing the number of the matched amino acid residues by the number of the total amino acid residues. When the above-mentioned alignment is carried out, a gap(s) is/are appropriately inserted into one or both of the two sequences to be compared as required. Such alignment of sequences can be easily carried out using a well-known program such as BLAST, FASTA or CLUSTAL W. In cases where a gap(s) is/are inserted, the above-mentioned number of the total amino acid residues is calculated by counting one gap as one amino acid residue. When the thus counted numbers of the total amino acid residues are different between the two sequences to be compared, the identity (%) is calculated by dividing the number of the matched amino acid residues by the number of the total amino acid residues of the longer sequence.

The 20 types of amino acids which constitute naturally-occurring proteins can be classified into groups according to the similar property, for example, into the following groups: neutral amino acids with side chains having low polarity (Gly, Ile, Val, Leu, Ala, Met, Pro); neutral amino acids having hydrophilic side chains (Asn, Gln, Thr, Ser, Tyr, Cys); acidic amino acids (Asp, Glu); basic amino acids (Arg, Lys, His); and aromatic amino acids (Phe, Tyr, Trp). It is known that, in most cases, substitution of an amino acid(s) within the same group does not change the properties of a polypeptide. Accordingly, in cases where a small number of amino acid residues in SEQ ID NO:2 are substituted with amino acid residues within the same group, it is especially likely that the reactivity of such a polypeptide with an antibody induced in a living body against pp28 produced by HCMV is at similar level to that of a polypeptide composed of the original amino acid sequence of SEQ ID NO:2. A polypeptide composed of an amino acid sequence substituted in the above-described manner is one preferred example of the polypeptide which may be used in the present invention whose amino acid sequence is different from SEQ ID NO:2.

The term "artificial polypeptide" means a polypeptide which is artificially produced by a chemical synthesis, genetic engineering technique or any other method, and does not include one which is obtained by collecting a protein expressed and produced from HCMV genome in cells infected with HCMV. As described above, the pp28 amino acid sequence and the base sequence encoding it are known as registered in the database, and described also in SEQUENCE LISTING of the present application. Further, since the codons encoding each amino acid are known, the base sequence of a polynucleotide encoding a specific amino acid sequence can be easily specified. Therefore, even in the case of a polypeptide which is composed of an amino acid sequence different from SEQ ID NO:2, the base sequence encoding such a polypeptide can be easily specified. A polypeptide to be used as an antigen in the present invention may be produced by any method based on such sequence information.

Specific examples of the chemical synthesis method include the Fmoc method (fluorenylmethyloxycarbonyl method), the tBoc method (*t*-butyloxycarbonyl method) and the like. Synthesis may also be carried out by a conventional method using any of various types of commercially available peptide synthesizers. In the case of chemical synthesis, a desired polypeptide can be synthesized based only on the amino acid sequence.

Methods for producing a polypeptide by a genetic engineering technique are also well known. Briefly, an antigen polypeptide of interest may be obtained as a recombinant polypeptide by preparing a polynucleotide encoding the above-described polypeptide, incorporating the polynucleotide into an appropriate expression vector, expressing it using an appropriate expression system, and collecting and purifying the expressed polypeptide. The polypeptide to be used as an antigen in the present invention has a size of not less than a hundred and several tens of residues. Therefore, in order to synthesize in a large scale at a low cost, it is preferred to produce the recombinant polypeptide by a genetic engineering technique using host cells such as *Escherichia coli.* Depending on the type of the host cells to be used, the recombinant polypeptide may be modified by various types of post-translational modifications (elimination of N-terminal methionine, N-terminal acetylation, glycosylation, limited degradation by an intracellular protease, myristoylation, isoprenylation, phosphorylation, etc.). Such polypeptides in the post-translationally modified form may also be used as an antigen in the method of the present invention, as long as they have the reactivity with an antibody induced in a living body against pp28 produced by HCMV.

In the case where a polypeptide composed of the amino acid sequence shown in SEQ ID NO:2 is produced by a genetic engineering technique, the polynucleotide (SEQ ID NO:1) encoding the said polypeptide may be prepared, for example, by infecting appropriate host cells with HCMV which has pp28 composed of the amino acid sequence shown in SEQ ID NO:2 (e.g. AD 169 strain), extracting viral DNA from the infected cells, and synthesizing DNA by PCR. Those skilled in the art can easily design and prepare primers to be used in the PCR based on the base sequence shown in SEQ ID NO:1. For example, the primers composed of the base sequences shown in SEQ ID NOs:3 and 4, which were used in the Examples, may be used. If an HCMV strain which expresses pp28 composed of any other amino acid sequence is used, then a polynucleotide which encodes a polypeptide composed of an amino acid sequence different from SEQ ID NO:2 can be prepared. In addition, a polynucleotide which encodes a polypeptide composed of a desired amino acid sequence may be obtained by using a commercially available nucleic acid synthesizer, or by arbitrarily introducing a mutation(s) in accordance with a conventional method into DNA prepared in the above-described manner. A desired polypeptide may be obtained by incorporating the thus-prepared polynucleotide into an appropriate vector, expressing it in an appropriate expression system, and collecting and purifying the expressed polypeptide. Vectors and various types of expression systems (for example, bacterial expression systems, yeast cell expression systems, mammalian cell expression systems, insect cell expression systems, cell-free expression systems and the like), which may be used, are also well known, and various vectors, host cells, reagents and kits are commercially available. Hence, those skilled in the art can appropriately select therefrom to use them. An HCMV strain can also be easily obtained, as HCMV strains are commercially available and also can be obtained by separating it from an infected patient. The individual techniques *per se* to extract viral DNA, to carry out PCR, to incorporate DNA into a vector, to introduce a vector into a host cell, to collect and purify an expressed polypeptide, and the like are well-known conventional methods.

As will be concretely described in the Examples below, a recombinant polypeptide composed of the amino acid sequence shown in SEQ ID NO:2 can be expressed using an *Escherichia coli* expression system, and the expressed polypeptide can be easily purified using, for example, an antibody column on which a monoclonal antibody specifically binding to pp28 is immobilized. By using the thus obtained recombinant polypeptide as an antigen for immunoassay, all HCMV-infected patients can be detected without fail. A method for producing a monoclonal antibody is a well-known conventional method, and those skilled in the art can easily produce a monoclonal antibody. For example, as will also be described in the Examples below, a monoclonal antibody which specifically binds to pp28 may be obtained by immunizing an animal with an inactivated virus, collecting antibody-producing cells such as spleen cells from the animal, fusing them with myeloma cells to produce hybridomas, selecting a hybridoma which produces an antibody having a desired binding ability, allowing it to proliferate, and collecting the monoclonal antibody from the culture supernatant.

Immunoassays *per se* are well known in the art. When classified based on the reaction mode, immunoassays can be classified into sandwich methods, competition methods, agglutination methods, immunochromatography, Western blotting or the like; and, when classified based on the label, immunoassays can be classified into radioimmunoassays, fluoroimmunoassays, enzyme immunoassays (EIA), biotin immunoassays or the like. Various techniques for antibody testing in which an antigen is used are also known, and specific examples thereof include, but not limited to, EIA (such as ELISA, CLEIA (chemiluminescent enzyme immunoassay), Western blotting), agglutination methods (such as a latex agglutination method), complement-fixation reactions (CF) and the like. When an anti-pp28 antibody in a sample is measured according to the method of the present invention, any of known immunoassays may be used. It is noted here that, in the present invention, the term "measure/measurement" includes detection, quantification and semi-quantification.

When used in an immunoassay, an artificial polypeptide antigen described above may be used in a form in which an arbitrary amino acid sequence(s) is/are added to one terminus or both termini of the above-mentioned artificial polypeptide (i.e. may be used as a polypeptide composed of an amino acid sequence containing such an additional sequence(s)). It is a widely known fact in the art that even a fusion protein comprising different proteins can be detected with an antibody which recognizes any one of the different proteins. Therefore, an anti-pp28 antibody in a sample can also be measured by immunoassay even when the above-described artificial polypeptide is used in such a form. For example, even in cases where an artificial polypeptide is used in a form of a fusion protein with other protein(s), it is the artificial polypeptide moiety that serves as an antigen for an anti-pp28 antibody in the fusion protein, and therefore such cases are included in "use an artificial polypeptide as an antigen". Thus, the cases where an artificial polypeptide antigen described above is used in a form in which an arbitrary amino acid sequence(s) is/are added to one terminus or both termini of said artificial polypeptide are also within the scope of the method of the present invention. The amino acid sequence which may be added to the artificial polypeptide may be a sequence which constitutes any functional protein or functional fragment thereof, or may be a sequence which is not physiologically active such as a linker. In cases of a recombinant polypeptide, addition of a tag sequence such as GST or His may occur in the production process, and such a polypeptide which still comprises a tag sequence added thereto can also be used as an antigen.

For example, in the case of ELISA or CLEIA, an artificial polypeptide antigen described above is immobilized on the solid phase of a plate, particle or the like, and the solid phase is reacted with a sample to capture an anti-pp28 antibody in the sample on the solid phase. After washing, the solid phase is reacted with an enzyme-labeled anti-IgG antibody and/or anti-IgM antibody, and thereafter, the solid phase is washed and a substrate is added thereto. Thus, the anti-pp28 antibody captured on the solid phase can be measured based on the amount of the enzyme reaction. In the case of Western blotting, the measurement may be carried out by electrophoresing an artificial polypeptide antigen described above; transferring it to a membrane; reacting the membrane with a sample; and then performing, in the same manner as described above, a reaction with an enzyme-labeled anti-IgG antibody and/or anti-IgM antibody. In the case of an agglutination method, the measurement may be carried out, for example, by immobilizing an artificial polypeptide antigen described above on latex particles or the like; reacting the latex particles or the like with a sample; and measuring the amount of the agglutinated particles by absorbance or the like.

The major antibodies induced in a living body against HCMV are IgG and IgM. In cases of primary infection, IgM first increases, reaches a peak in several days after the infection, and decreases thereafter; and, IgG starts to increase in about 1 week after the infection, and thereafter continues to be present for a long period of time. Therefore, when carrying out a technique by which anti-pp28 IgG and IgM in a sample can be detected separately from each other (such as ELISA or Western blotting mentioned above), it is preferred, from the viewpoint of surely detecting HCMV infection, that both of an anti-IgG antibody and an anti-IgM antibody be used in detecting the anti-pp28 antibody captured by an antigen.

The sample to which the method of the present invention is applied is a sample separated from a subject, preferably a blood sample (such as whole blood, plasma, serum).

In carrying out the method of the present invention, an HCMV antigen protein other than the above-described artificial polypeptide, or a fragment thereof may be used at the same time. For example, in the case of ELISA, the individual antigens may be all mixed up and then immobilized on a plate, or the antigens may be each separately immobilized in wells, respectively, to react the plate or wells with a sample. In the case of CLEIA, the individual antigens may all be mixed up and then immobilized on particles to react the particles with a sample. In the case of Western blotting, the individual antigens may be, for example, all mixed up and then electrophoresed, and thereafter transferred to a membrane, followed by reacting the membrane with a sample. In the case of an agglutination method, the individual antigens may be all mixed up and then immobilized on particles, or the individual antigens may be each separately immobilized on particles and then the particles may be mixed, and the thus prepared particles may be reacted with a sample.

An artificial polypeptide as described above may be provided as a reagent for detecting HCMV infection. The reagent may contain only the above-described polypeptide, or may further contain one or more antigen proteins other than pp28, or fragments thereof. In addition, the reagent may further contain any other component(s) useful for stabilization and/or the like of these polypeptides. Furthermore, the reagent may be provided also in a form in which an antigen polypeptide(s) is/are immobilized on the solid phase such as a plate, particle or the like.

The above-described detection reagent may be provided as a kit for detecting HCMV infection as appropriate in combination with other reagents and/or the like. Other reagents necessary for immunoassay are well known. For example, in addition to the above-described detection reagent, the detection kit of the present invention may comprise a buffer solution which can be used as a sample diluent, a washing solution and/or the like; a labeled anti-immunoglobulin antibody for detecting an antibody bound to an antigen polypeptide; and/or the like.

### EXAMPLES

The present invention will now be described more concretely by way of Examples. However, the present invention is not limited to the Examples below.

### 1. Selection of Antigen Candidates

Among HCMV proteins, 15 kinds of proteins (see, Table 1 below) were synthesized in full-length forms, respectively, by using a cell-free wheat germ expression system. For the synthesis, Protemist DT from CellFree Sciences Co., Ltd. was used. Insert DNAs to be inserted into a vector were obtained by infecting MRC-5 cells with HCMV AD169 strain (purchased from RIKEN BioResource Center), extracting viral DNA from the infected cells, and amplifying DNAs encoding each full length protein by PCR using a commercially available kit. Each of the amplified DNAs was cloned into a plasmid vector containing the SP6 promoter, and using the obtained plasmid DNAs, the transcription reaction at 37°C for 6 hours and the translation at 15°C for 20 hours were carried out in 5 mL reaction system (plasmid DNA: 25 µg), thereby synthesizing the respective full length proteins. For the amplification of DNA of pp28, the primers composed of the base sequences shown in SEQ ID NOs:3 and 4 (containing the BamHI recognition sequence and the NotI recognition sequence, respectively) were used; and the PCR was performed using Pfu Ultra DNA plymerase (Agilent Technologies) under the reaction conditions of: a denaturation treatment of 95°C for 2 minutes; 30 cycles of 95°C for 20 seconds, 55°C for 20 seconds and 72°C for 1 minute; and a final treatment of 72°C for 3 minutes.

**[Table 1]**

| Protein Name | Number of Amino Acids |
|---|---|
| ppUL32 (pp150) | 1048 |
| ppUL44 (pp52) | 433 |
| ppUL83 (pp65) | 561 |
| ppUL80a (pp38) | 373 |
| ppUL99 (pp28) | 190 |
| ppUL55 (gp130) | 907 |
| UL75 | 743 |
| UL123 | 491 |
| UL122 | 411 |
| pUL57 | 1235 |
| UL115 | 306 |
| UL100 | 372 |
| UL73 | 138 |
| UL74 | 466 |
| UL82 | 559 |

### 2. Determination of Candidate Antigens

The respective full length proteins synthesized above were examined for the reactivity with 100 sera from HCMV-infected patients by Western blotting. The detection was carried out by electrophoresing each full length protein, transferring it to a membrane, reacting the membrane with 100 HCMV-infected patients' sera, and then carrying out detection with anti-Human IgG + IgM. As a result, three types of proteins, that is, pp150, pp28 and gp130, exhibited a reactivity of 100%, as shown in Table 2 below.

**[Table 2]**

| Result of 100 HCMV-infected Patients' Sera | | |
|---|---|---|
| | Number Of Positive Sera | % |
| ppUL32 (pp150) | 100 | 100 |
| ppUL44 (pp52) | 52 | 52 |
| ppUL83 (pp65) | 67 | 67 |
| ppUL80a (pp38) | 65 | 65 |
| ppUL99 (pp28) | 100 | 100 |
| ppUL55 (gp130) | 100 | 100 |
| UL75 | 25 | 25 |
| UL123 | 65 | 65 |
| UL122 | 67 | 67 |
| pUL57 | 27 | 27 |
| UL115 | 2 | 2 |
| UL100 | 95 | 95 |
| UL73 | 1 | 1 |
| UL74 | 0 | 0 |
| UL82 | 18 | 18 |

### 3. Production of Antigen by Escherichia coli Expression System

In order to produce a recombinant protein in a large scale, it is desirable to use an *Escherichia coli* expression system. In this study, the full length production of three proteins which exhibited a 100% reactivity was investigated using a conventional *Escherichia coli* expression system. Each of the above-prepared cDNAs which encoded the respective antigen proteins was incorporated into an expression vector for *Escherichia coli,* and the obtained vectors were introduced into *Escherichia coli* and expressed. As a result, expression of pp28 could be observed. On the other hand, pp150 and gp130 (a membrane protein) could not be expressed in *Escherichia coli.*

Purification of pp28 which could be expressed in *Escherichia coli* was carried out using an antibody column. The antibody column for the purification was prepared as follows: that is, a mouse was immunized with an inactivated virus to produce a pp28 antigen-specific monoclonal antibody in accordance with a conventional method, and the obtained antibody was immobilized on a CNBr-Activated Sepharose 4B column (GE Healthcare). As a result, pp28 protein could be purified by only one round of column treatment without contamination by proteins derived from *Escherichica coli* (Fig. 1). By using this recombinant pp28 protein, 100% of the HCMV-infected patients could also be detected.

## Claims

1. A method for detecting human cytomegalovirus infection, comprising measuring an antibody against pp28 which may be contained in a sample separated from a subject by immunoassay using as an antigen an artificial polypeptide composed of an amino acid sequence having an identity of not less than 90% to the amino acid sequence shown in SEQ ID NO:2, said artificial polypeptide having a reactivity to bind by antigen-antibody reaction to an antibody induced in a living body against pp28 produced by human cytomegalovirus.

2. The method according to claim 1, wherein said artificial polypeptide is composed of the amino acid sequence shown in SEQ ID NO:2 or an amino acid sequence which is the same as that shown in SEQ ID NO:2 except that one or several amino acids are substituted, deleted, inserted and/or added.

3. The method according to claim 2, wherein said artificial polypeptide is composed of the amino acid sequence shown in SEQ ID NO:2.

4. The method according to any one of claims 1 to 3, wherein said artificial polypeptide is a recombinant polypeptide produced by a genetic engineering technique.

5. A reagent for detecting human cytomegalovirus infection, comprising an artificial polypeptide composed of an amino acid sequence having an identity of not less than 90% to the amino acid sequence shown in SEQ ID NO:2, said artificial polypeptide having a reactivity to bind by antigen-antibody reaction to an antibody induced in a living body against pp28 produced by human cytomegalovirus.

6. A kit for detecting human cytomegalovirus infection, comprising the reagent according to claim 5.
